(19) Europäisches Patentamt
European Patent Office
Office européen des brevets

(11) Veröffentlichungsnummer: **0 073 465**
**B1**

(12) # EUROPÄISCHE PATENTSCHRIFT

(45) Veröffentlichungstag der Patentschrift: **05.02.86**

(21) Anmeldenummer: **82107788.0**

(22) Anmeldetag: **25.08.82**

(51) Int. Cl.[4]: **C 07 C 172/00**, A 61 K 31/59, C 07 J 9/00, C 07 J 17/00, C 07 J 31/00, C 07 J 51/00, C 07 C 31/42, C 07 C 43/12, C 07 C 69/63, C 07 C 143/68, C 07 D 307/32

(54) **Cholecalciferolderivate.**

(30) Priorität: **28.08.81 US 297446**

(43) Veröffentlichungstag der Anmeldung: **09.03.83 Patentblatt 83/10**

(45) Bekanntmachung des Hinweises auf die Patenterteilung: **05.02.86 Patentblatt 86/6**

(84) Benannte Vertragsstaaten: **AT BE CH DE FR GB IT LI LU NL SE**

(56) Entgegenhaltungen:
**FR - A - 2 453 151**
**US - A - 4 201 881**

**CHEMICAL ABSTRACTS, Band 93, Nr. 25, 22. Dezember 1980, Seite 893, Zusammenfassung Nr. 239788v COLUMBUS OHIO (US)**
**TETRAHEDRON LETTERS, Nr. 22, Mai 1979 Pergamon Press OXFORD (GB) Y. KABAYASHI et al.: "Synthesis of 24, 24-difluoro-and 24-fluoro-25-hydroxy vitamin D3", Seiten 2023-2026**

(73) Patentinhaber: **F. HOFFMANN-LA ROCHE & CO. Aktiengesellschaft, CH-4002 Basel (CH)**

(72) Erfinder: **Partridge, John Joseph, 121 Norwood Avenue, Upper Montclair, N.J.07043 (US)**
Erfinder: **Shiuey, Shian-Jan, 331 Bloomfield Avenue, Nutley, N.J. 07110 (US)**
Erfinder: **Uskokovic, Milan Radoje, 253 Highland Avenue, Upper Montclair, N.J. 07043 (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 073 465 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Cholecalciferolderivate der Formel

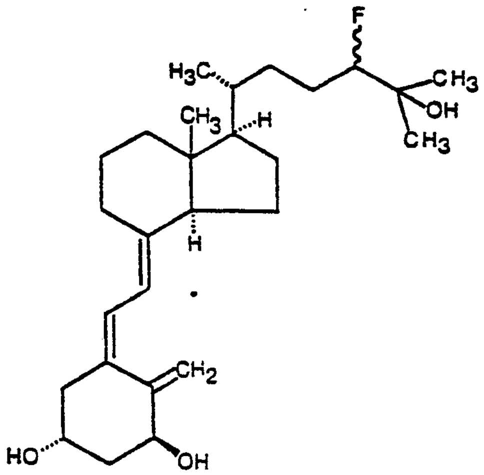

I

Die Erfindung betrifft ferner pharmazeutische Präparate auf der Basis der Verbindungen der Formel I, ein Verfahren zur Herstellung dieser Verbindungen und neue in diesem Verfahren vorkommende Zwischenprodukte.

Nach dem Verfahren der vorliegenden Erfindung werden die Verbindungen der Formel I dadurch hergestellt, dass man ein Precholecalciferolderivat der Formel

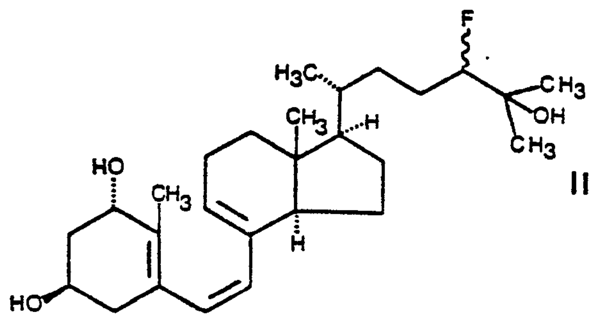

IIA

thermisch isomerisiert.

In den Formeln bringt eine Wellenlinie zwischen einem C-Atom und einem Substituenten zum Ausdruck, dass die absolute Konfiguration am C-Atom R oder S ist oder dass die entsprechende Verbindung in Form eines R,S-Gemisches vorliegt.

Die thermische Isomerisierung einer Verbindung der Formel IIA kann durch Erhitzen in einem inerten Losungsmittel wie einem Aether, z.B. Dioxan, Tetrahydrofuran oder Dimethoxyäthan; einem aromatischen Kohlenwasserstoff, z.B. Benzol oder Toluol; unter einer inerten Atmosphäre, wie Argon oder Helium; in an sich bekannter Weise, z.B. wie beschrieben in J.A.C.S. 98 (1973) 2748, durchgeführt werden.

Im Rahmen der Erfindung bezieht sich der Ausdruck 'nieder' auf Reste mit 1-8 Kohlenstoffatomen. Beispiele von Niederalkylgruppen sind Methyl, Aethyl, n-Propyl, i-Propyl, tert.-Butyl, Hexyl, Heptyl und Octyl. Beispiele von Niederalkylengruppen sind Methylen, Aethylen und Propylen. Beispiele von Niederalkoxygruppen sind Methoxy, Aethoxy, Isopropoxy und tert.-Butoxy. Beispiele von Niederalkanoyloxygruppen sind Formyloxy, Acetoxy, Butyryloxy und Hexanoyloxy. Der Ausdruck 'substituiert' bezogen auf 'phenyl' betrifft Phenylgruppen die mit einer oder mehreren Gruppen, wie Niederalkyl, Fluor, Chlor, Brom, Jod, Nitro, Cyan oder Trifluormethyl, substituiert sind.

Die Verbindungen der Formel II-A können dadurch hergestellt werden, dass man eine Verbindung der Formel

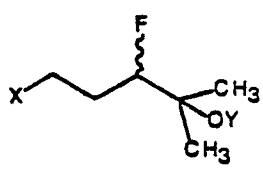

III

worin X Jod, Brom, Chlor, Niederalkylsulfonyloxy, Phenylsulfonyloxy oder substituiertes Phenylsulfonyloxy und Y eine Gruppe der Formel

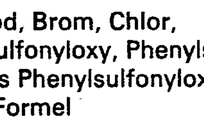

worin $R^1$ Wasserstoff oder Niedralkyl, $R^2$ und $R^3$ Niederalkyl oder $R^2$ und $R^3$ zusammen $C_{3-6}$-Alkylen darstellen,

mit einem metallierten Pregn-5-en-21-carbonsäureester der Formel

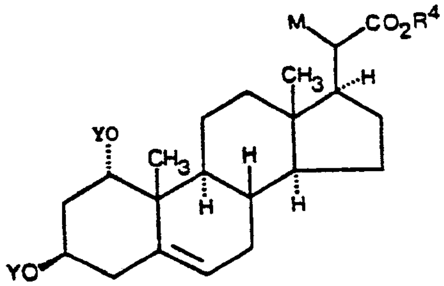

IV A

worin M Lithium Natrium Kalium, Magnesium/2 oder Zink/2 R Niederalkyl Phenyl oder substituiertes Phenyl darstellt und Y die obige Bedeutung hat,

nach der in J. Chem. Soc., Perkin Trans. I, 1282 (1978) beschriebenen Methode umsetzt. Man erhält eine alkylierte Verbindung der Formel

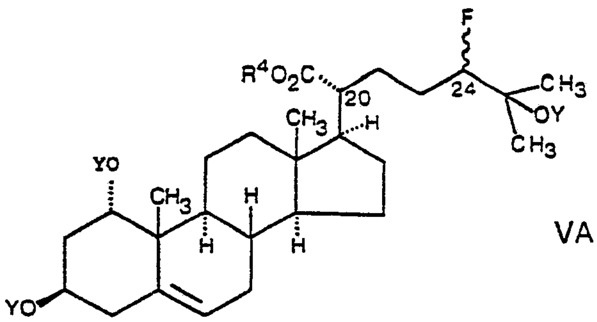

VA

worin $R^4$ und Y die obige Bedeutung haben.

Diese Reaktion kann in einem inerten aprotischen Lösungsmittel, wie einem Aether, z.B. Tetrahydrofuran, oder einem Amid, z.B. Hexamethylphosphoramid, zweckmässig bei einer Temperatur zwischen -78 und 60°C durchgeführt werden. Das Prcdukt der Formel V-A mit der erwünschten absoluten 20R-Konfiguration kann durch die üblichen chemischen und physikalischen Methoden isoliert werden.

Die Verbindung der Formel V-A wird dann zu einem Alkohol der Formel

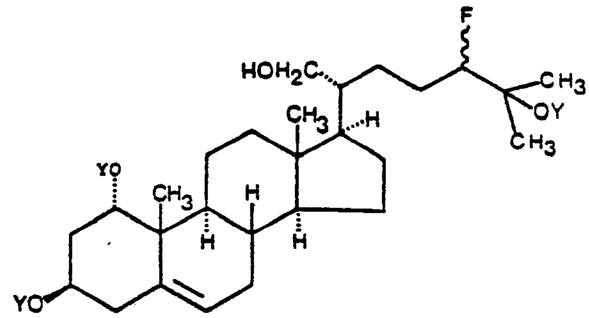

VB

worin Y die obige bedeutung hat, reduziert. Beispiele von komplexen Metallhydriden die bei dieser Reduktion verwendet werden können sind Alkalimetallaluminiumhydride, z.B. Lithiumaluminiumhydrid; mono-, di- oder tri(Niederalkoxy)alkalimetallaluminiumhydride, z.B. Lithium tris(tert.-Butoxy)aluminiumhydrid; ein mono-, di- oder tri(Niederalkoxy-niederalkoxy)alkalimetallaluminiumhydrid, z.B. Natrium bis(2-Methoxyäthoxy)aluminiumhydrid; oder ein di-Niederalkylaluminiumhydrid, z.B. Diisobutylaluminiumhydrid. Bei der Reduktion können als Lösungsmittel Aether, wie Diäthyläther; verwendet werden. Die Reduktion wird zweckmässig bei einer Temperatur zwischen etwa 0 und 100°C durchgeführt.

Der Alkohol der Formel V-B wird dann in an sich bekannter Weise zum Halogenid oder Sulfonat der Formel

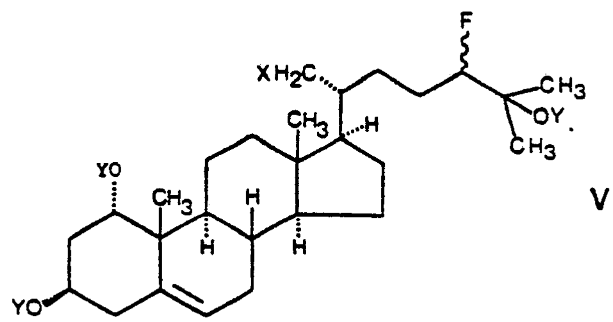

V C

worin X und Y die obige Bedeutung haben, umgewandelt.

Zur Herstellung eines Sulfonats der Formel V-C wird die Verbindung der Formel V-B in Gegenwart von einer Base mit einem Sulfonylhalogenid umgesetzt. Die Herstellung eines Halogenids der Formel V-C kann dadurch bewerkstelligt werden, dass man entweder einen Alkohol der Formel V-B mit einem Halogenierungsmittel, wie Phosphortribromid, umsetzt, oder einen

Sulfonatester der Formel V-C, z.B. ein Tosylat, mit einer ein Halogenion enthaltenden Verbindung, wie einem Alkalimetallbromid oder -jodid, z.B. Kaliumbromid, umsetzt.

Die Verbindung der Formel V-C wird dann zu einer Verbindung der Formel

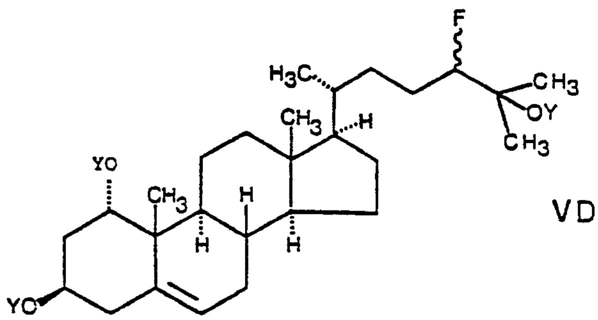

VD

worin Y die obige Bedeutung hat, mittels eines komplexen Metallhydrids reduziert.

Die Reduktion wird zweckmässig mit einem der Reduktionsmittel und in einem der Lösungsmittel, die weiter oben in Zusammenhang mit der Reduktion einer Verbindung der Formel V-A zu einer Verbindung der Formel V-B erwähnt wurden, durchgeführt. Die Reaktionstemperatur liegt zwischen etwa Raumtemperatur und 100°C. Weitere bei der Reduktion verwendbare Reduktionsmittel, insbesondere wenn X Jod oder Brom darstellt, sind Alkalimetallcyanborhydride, wie 'Natriumcyanborhydrid (Natriumcyantrihydroborat); tri(Niederalkyl)zinnhydrid, wie tri-n-Butylzinnhydrid; und tri(Aryl)zinnhydride, wie Triphenylzinnhydrid. Die Reduktion wird zweckmässig bei einer Temperatur zwischen etwa -20 und 80°C durchgeführt.

Die Verbindung der Formel V-D wird dann durch Abspaltung der Schutzgruppen Y mittels einer starken Säure in einem protischen Lösungsmittel in eine Verbindung der Formel

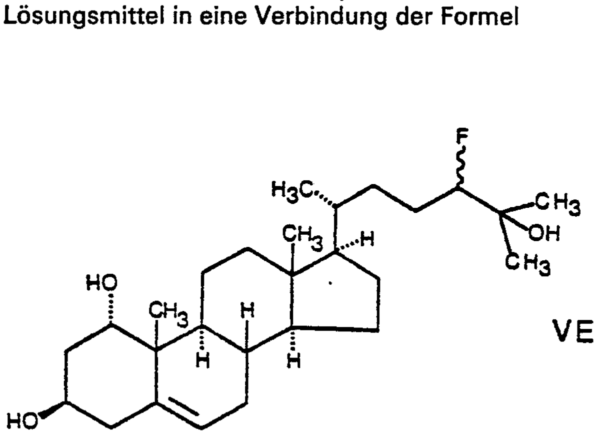

VE

umgewandelt. Als starke Säure können Mineralsäuren wie Salzsäure, und organische Sulfonsäuren, wie p-Toluolsulfonsäure, verwendet werden. Als Lösungsmittel können Alkohole, wie Methanol, und Wasser mit einem mischbaren Cosolvent zur Solubilisierung der organischen Reaktanten, wie einem Aether, z.B. Tetrahydrofuran; oder einem Keton, wie Aceton, verwendet werden. Die Reaktion wird zweckmässig bei einer Temperatur zwischen etwa -10 und 80°C durchgeführt.

DAS Cholesterin der Formel V-E wird dann in an sich bekannter Weise zu einem 1α,3β-Dialkanoat der Formel

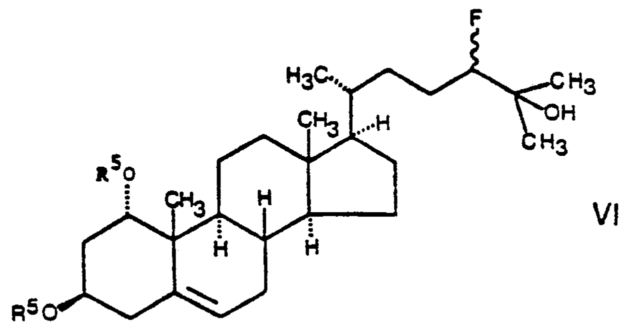

VI A

worin $R^5$ Niederalkanoyl darstellt, alkanoyliert.

Die Verbindung der Formel VI-A wird einer allylischen Halogenierung unterworfen. Man erhält ein Gemisch von 7α- und 7β-Halocholesterinen der Formel

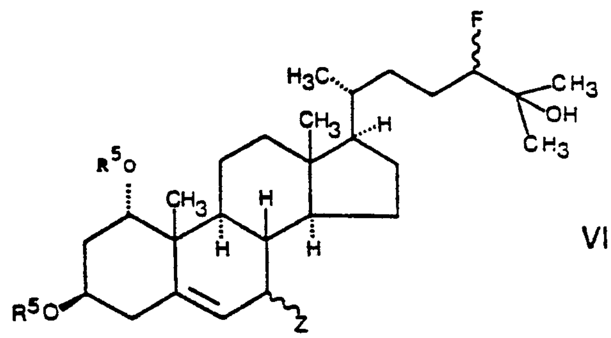

VIB

worin $R^5$ die obige Bedeutung hat und Z Jod, Brom oder Chlor darstellt.

Die Reaktion wird mit einem Halogenierungsmittel, wie N-Chlor-oder N-Bromsuccinimid, gelöst in einem gesattigten aliphatischen Kohlenwasserstoff oder Halokohlenwasserstoff, wie Hexan, in Gegenwart eines Neutralisationsmittels, wie Natriumbicarbonat, beim Siedepunkt des Reaktionsgemisches durchgeführt.

Ohne Trennung des 7β-Haloisomeren von dem überschüssigen 7α-Isomeren wird die Verbindung

der Formel VI-B mittels eines heteroaromatischen tertiären Amins, wie eines Pyridin, z.B. eines Picolins, Lutidins oder Collidins; oder eines aliphatischen tertiären Amins, z.B. Triäthylamin, Tripropylamin, 1,5-Diazabicyclo(4.3.O)non-5-en oder 1,4-Diazabicyclo(2.2.2)octan, zu einem Steroid-5,7-dien der Formel

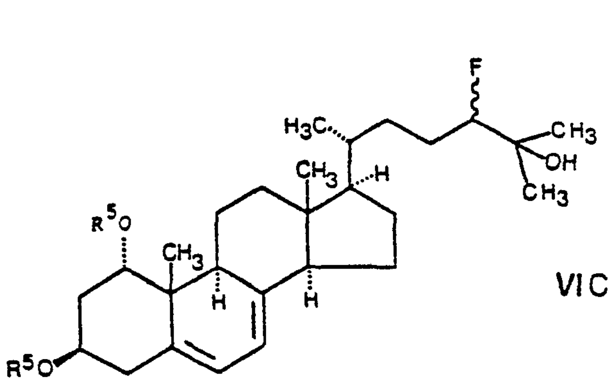

VI C

worin $R^5$ die obige Bedeutung hat, dehydrohalogeniert. Trialkylphosphite können auch in der Dehydrohalogenierungsstufe verwendet werden. Die Reaktion wird zweckmässig in einem inerten organischen Lösungsmittel, wie einem aromatischen oder aliphatischen Lösungsmittel, z.B. Xylol, bei einer Temperatur zwischen etwa 50° C und der Rückflusstemperatur des Reaktionsgemisches durchgeführt. Das Steroid-5,7-dien der Formel VI-C kann in an sich bekannter Weise durch chemische und physikalische Methoden, wie Chromatographie, isoliert werden und auf diese Weise von unerwünschten Unreinigkeiten, wie dem entsprechenden 4,6-Dien, getrennt werden.

Das Steroid 5 7-Dien der Formel VI-C wird in ein Precholecalciferol-1α,3β-dialkanoat der Formel

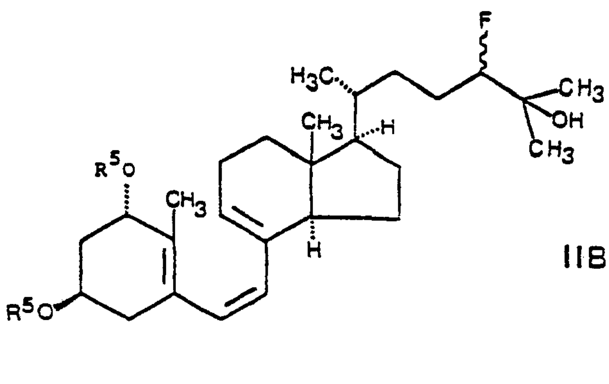

IIB

worin $R^5$ die obige Bedeutung hat, durch Bestrahlung unter einer inerten Atmosphäre, wie Stickstoff, bei einer Temperatur zwischen etwa -40 und +25° C übergeführt. Als Bestrahlungsquellen werden zweckmässig Hoch- oder Niederdruckgquecksilberlampen verwendet. Zweckmässig werden bei der Bestrahlung inerte organische Lösungsmittel, wie Gemische von gesättigten aliphatischen Kohlenwasserstoffe und ätherischen Lösungsmitteln, z.B. Gemische von Hexan und Tetrahydrofuran, verwendet.

Nach der Bestrahlung werden die Lösungsmittel durch Eindampfen entfernt und der Rückstand wird in reines precholecalciferol-1α,3β-dialkanoat der Formel II-B und reines unverändertes Steroid 5,7-Dien der Formel VI-C durch Flüssigchromatographie, mittels eines festen Absorptionsmittels, wie eines aus Glaskügelchen bestehenden Materials, und eines inerten organischen Eluierungsmittels aufgetrennt. Als Eluierungsmittel werden zweckmässig Gemische von Kohlenwasserstoffen und Estern, wie ein Gemisch von n-Hexan und Aethylacetat, verwendet.

Unverändertes 4,7-Dien der Formel VI-C wird erneut der Bestrahlung unterworfen und man erhält zusätzliche Mengen an reinem Precholecalciferol-1α,3β-dialkanoat der Formel II-B.

Die Verbindung der Formel II-B wird dann mittels einer starken Base in einem protischen Lösungsmittel zu einer Verbindung der Formel II-A verseift. Als Base werden zweckmässig Alkali- oder Erdalkalimetallhydroxide oder Alkoxide, wie Kaliumhydroxid, verwendet. Als Lösungsmittel werden zweckmässig Alkohole, wie Methanol, und Wasser mit einem mischbaren Cosolvent zur Solubilisierung der organischen Reaktanten, wie einem Aether, z.B. Tetrahydrofuran, verwendet. Die Abspaltung der Alkanoylgruppen $R^5$ wird vorzugsweise bei einer Temperatur zwischen etwa -20 und 60° C und unter einer inerten Atmosphäre wie Stickstoff durchgeführt.

Die Verbindungen der Formel III können wie im folgenden Schema, worin $R^5$ und Y die obigen Bedeutungen haben, und wie in den Beispielen beschrieben hergestellt werden.

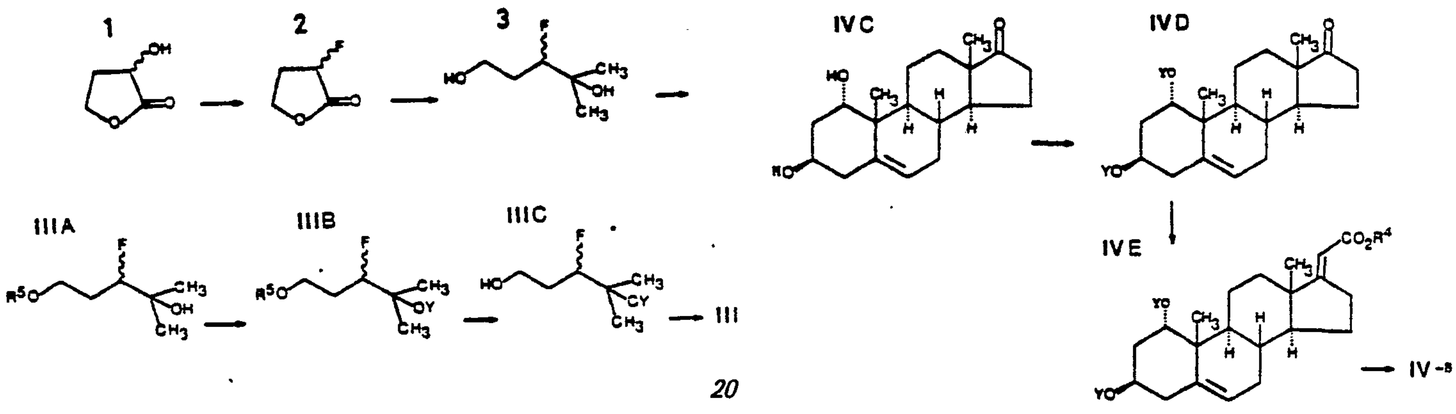

Die metallierten Verbindungen der Formel IV-A können durch Behandlung einer Verbindung der Formel

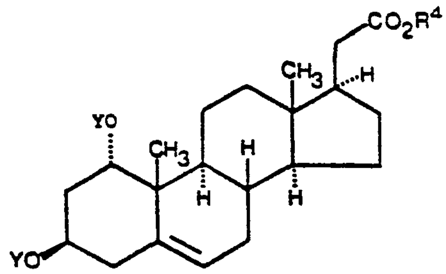

IV B

worin $R^4$ und Y die obige Bedeutung haben, mit einem geeigneten organometallischen Reagens, wie z.B. Lithiumdiisopropylamid, Natriumhexamethyldisilasan oder Kaliumhydrid, hergestellt werden.

Die Verbindungen der Formel IV-B können wie in dem folgendem Schema, worin Y und $R^4$ die obige Bedeutung haben, und wie in den Beispielen beschrieben, hergestellt werden.

Die Verbindungen der Formeln II-A und II-B, d.h. der Formel

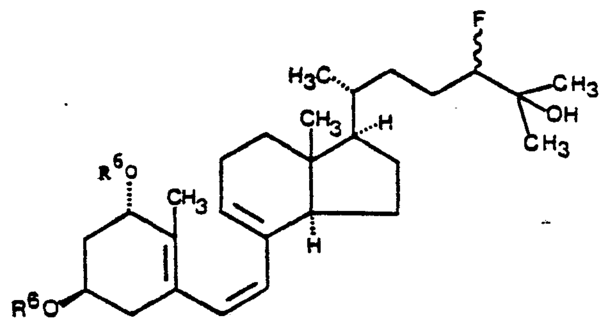

II

worin $R^6$ Wasserstoff oder Niederalkanoyl darstellt, diejenigen der Formeln VI-A, VI-B und VI-C, d.h. der Formel

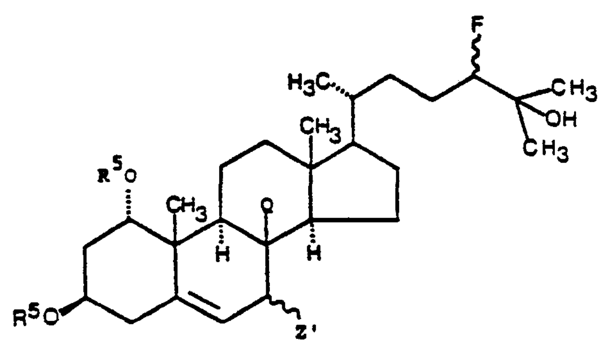

VI

worin Q Wasserstoff und Z' Wasserstoff, Jod, Brom oder Chlor, oder Q und Z' zusammen eine

zusätzliche 7,8-Bindung darstellen und $R^5$ die obige Bedeutung hat,

die jenigen der Formeln V-A, V-B, V-C, V-D und V-E, d.h. der Formel

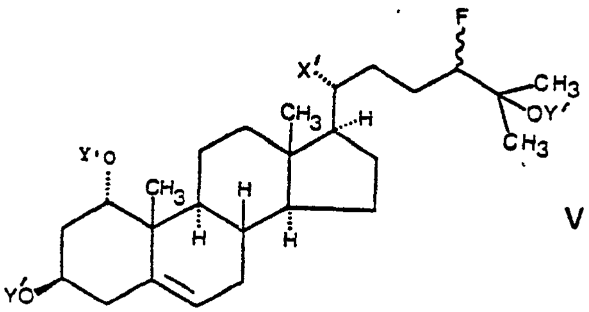

V

worin Y' Wasserstoff oder eine Gruppe Y und X' Methyl darstellen, oder Y' eine Gruppe Y und X' eine Gruppe $-CH_2X$, $-CH_2OH$ oder $-COOR^4$ darstellen, und Y, X und $R^4$ die obige Bedeutung haben,

diejenigen der Formeln IV-A und IV-B, d.h. der Formel

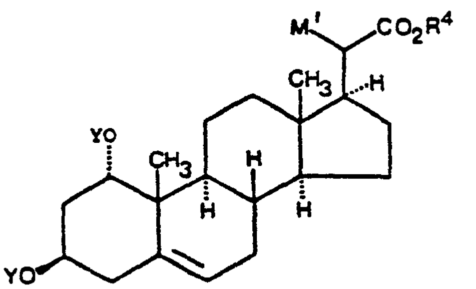

IV

worin M' Wasserstoff oder eine Gruppe M, und M, $R^4$ und Y die obige Bedeutung haben,

sowie diejenigen der Formeln 2, 3, III-A, III-B, III-C und III sind neu.

Die Verbindungen der Formel I sind dazu geeignet, den intestinalen Calciumtransport, den Calcium- und Phosphatspiegel im Serum, sowie die Anlagerung dieser Mineralien in den Knochen zu erhöhen. Diese Verbindungen können daher als Ersatz für das 1α,25-Dihydroxycholecalciferol, zur Behandlung von durch metabolische Calcium- und Phosphatmangelzustände gekennzeichnete Krankheiten Verwendung finden. Beispiele solcher Krankheiten sind Osteomalacia, Osteoporosis, Rachitis, Osteitis fibrosa cystica, renale Osteodystrophie, Osteosclerosis, Konvulsionen, Osteopenia, Fibrogenesis imperfecta ossium, sekundärer Hyperparathyroidismus, Hypoparathyroidismus, Hyperparathyroidismus, Cirrhosis, obstruktive Gelbsucht, drogeninduzierter Metabolismus, medullärer Karzinom, chronische renale Krankheiten, hypophosphatämische Vitamin D-resistente Rachitis, Vitamin D-abhängige Rachitis, Sarcoidosis, Glucocorticoidantagonismus, Missabsorptionssyndrom, Steatorrhea tropische Sprue, idiopatische Hypercalcämie und Milchfieber.

1α,25-Dihydroxy-24R-fluorcholecalciferol hat sich im anti-rachitogenischen Versuch bei Kücken als aktiv erwiesen. Ferner wurde eine mit der von 1α,25-Dihydroxycholecalciferol vergleichbaren, 24 Stunden andauernden Stimulierung der intestinalen Calciumabsorption bei einmaliger oraler Verabreichung von 100 Nanogramm iα,25Dihydroxy-24R-fluorcholecalciferol festgestellt.

Weissen Leghorn-Kücken wird unter UV-freier Beleuchtung eine Vitamin D-arme, 1% Calcium und 0,7% Phosphor enthaltende Diät gegeben. Die in Propylenglykol gelösten Testverbindungen werden 21 Tage den am Anfang des Versuchs 1 oder 2 Tage alten Kücken oral verabreicht. Kontrolltiere werden mit dem Vehikel behandelt. Die Kücken werden am Tag nach dem letzten Behandlungstag seziert und das Tibiaaschengewicht wird ermittelt. Es werden 9 oder 10 Kücken für jede Behandlungsgruppe und für die Kontrollgruppe verwendet. Die Resultate des antirachitogenischen Aktivitätsversuchs für 1α,25-Dihydroxy-24R-fluorcholecalciferol (Verbindung X) und für 24,24-Difluor-1α,25-dihydroxycholecalciferol (Verbindung A) sind in der Tabelle I wiedergegeben. Die Resultate zeigen das 1α,25-Dihydroxy24R-fluorcholecalciferol eine beträchtliche antirachitogenische Aktivität aufweist.

In einem weiteren Versuch werden den Kücken während 21 Tagen unter UV-freier Bestrahlung eine Vitamin D-arme Diät gegeben. Zur Bestimmung des Einflusses der Verbindung X auf die intestinale Calciumabsorption wird eine in Propylenglykol gelöste Einzeldosis oral verabreicht. Danach wird zu verschiedenen Zeitpunkten 2 Ci $^{45}Ca$ (Chlorid) oral verabreicht und die Serum Radioaktivität 45 Minuten nach Verabreichung des Isotops gemessen. In jeder Versuchsperiode werden Kontrolltiere verwendet, denen nur das Vehikel verabreicht wird. In jeder Behandlungsgruppe und jeder Kontrollgruppe werden 10 Kücken verwendet. Die Resultate sind in der Tabelle II angegeben. Sie zeigen, dass 1α,25-Dihydroxy-24R-fluorcholecalciferol während etwa 24 Stunden intestinale Calciumabsorption bewirkt.

**Tabelle I**

| Dosis ng/Kücken/Tag | Mittleres Tibiaaschengewicht (mg) Verbindung A | X |
|---|---|---|
| 0 | 120,9±8,3 | |
| 3 | 192,2± 6,2 | 293,3± 4,8 |
| 10 | 258,6±10,0 | 330,4± 7,7 |
| 30 | 229,8±14,2 | 337,9±10,6 |

**Tabelle II**

| Behandlung | Zeit (Stunden) | Serum $^{45}Ca$ cpm/0.2 ml |
|---|---|---|
| 0,2 ml Vehikel | 24 | 408±30 |
| Verbindung X | | 793±73 * |
| 0,2 ml Vehikel | 48 | 439±30 |
| Verbindung X | | 520±38 NS |
| 0,2 ml Vehikel | 72 | 398±31 |
| Verbindung X | | 372±39 NS |

* = statistisch signifikantes Resultat
NS = statistisch nicht signifikantes Resultat

1α,25-Dihydroxy-24R-fluorcholecalciferol kann in Tagesdosen von etwa 0,10-3,0, vorzugsweise 0,25-2,0 Mikrogramm bei solchen Krankheiten verabreicht werden, bei denen die Konzentration des endogen produzierten 1α,25Dihydroxycholecalciferols unter dem Normalwert liegt. Solche Krankheiten sind Osteodystrophie, durch Steroide induzierte Osteopenia, Hypoparathyroidismus, hypophosphatämische Rachitis und hypophosphatämische Osteomalacia. 1α,25-Dihydroxy-24R-fluorcholecalciferol kann oral, subcutan, intramuskulär, intravenös, intraperitonal oder topisch appliziert werden. Beispiele von pharmazeutischen Präparaten sind Tabletten, Kapseln oder Elixiere zur oralen Verabreichung, oder sterile Lösungen oder Suspensionen zur parenteralen Verabreichung. Diese pharmazeutischen Präparaten können folgende Hilfsstoffe enthalten: ein Bindemittel, wie Tragantgummi, Akazie, Maisstärke oder Gelatine; einen Excipienten, wie Calciumphosphat; ein Zersetzungsmittel, wie Mais oder Kartoffelstärke oder Alginsäure; ein Gleitmittel, wie Magnesiumstearat; ein Süsstoff, wie Sucrose, Lactose oder Saccharin; ein Geschmackmittel, wie pfefferminze, Wintergrün oder Sherryöl. Es können andere Materialien, wie Ueberzüge zur Modifizierung des Aspektes der präparate verwendet werden. Tabletten können z.B. mit Shellaczucker oder Weide überzogen werden. Ein Sirup oder Elixier kann die Aktivsubstanz, Sucrose als Süssmittel, Methyl- und Propylparaben als Konservierungsmittel, ein Farbstoff und einen Geschmacksstoff, wie Kirschen- oder Orangearoma, enthalten.

1α,25-Dihydroxy-24R-fluorcholecalciferol kann in Dosen von 100-1500 Mikrogramm/Tag zur Behandlung des Milchfiebers bei trächtigen Wiederkäuern in den üblichen Verabreichungsformen verwendet werden.

Sterile präparate zum Injizieren und/oder zur topischen Verabreichung können in üblicher Weise dadurch hergestellt werden, dass man 1α,25-Dihydroxy-25R-fluorcholecalciferol in einem Vehikel, wie einem 10-20% AethanolWassergemisch, einem 10-20% Propylenglykol-Wassergemisch, einem in der Natur vorkommenden pflanzlichen Oel, wie Sesamöl, Erdnussöl, Baumwollöl; oder einem synthetischen fetten Bindemittel, wie Aethyloleat, auflöst oder suspendiert. So enthält ein für die intravenöse Injection geeignetes Präparat 2-3 ml einer 10-20% Aethanol-Wasserlösung oder eines 10-20% propylenglykol-Wassergemisches und 200-1000 Mikrogramm 1α,25-Dihydroxy-24R-fluorcholecalciferol. Ein zur topischen Verabreichung geeignetes präparat besteht z.B. aus einer Lösung oder Suspension eines Pflanzenöls enthaltend 200-1000 Mikrogramm 1α,25-Dihydroxy-25R-fluorcholecalciferol. Zur oralen Verabreichung kann man auch 1α,25-Dihydroxy-24R-fluorcholecalciferol in Mengen von 100-1500 Mikrogramm in Fettsäurepillen verwendet werden.

Zur intramuskulären Injektion kann auch 1α,25-Dihydroxy-24R-fluorcholecalciferol in Mengen von 100-1500 Mikrogramm in einem Vehikel, wie einem Pflanzenöl, einer Aethanol-Wasserlösung oder einer Propylenglykol-Wasserlösung, enthaltend 80-95% Aethanol oder Propylenglykol, suspendiert werden.

**Beispiel 1**

A. Ein Gemisch von 0 104 g (0 00024 Mo1)[1α,3β,6Z,24R]24-fluor-9 10-secocholesta-5(10) 6,8-trien-1,3,25-triol und 80 mg p-Dioxan wird unter Rückfluss (100°C) während einer Stunde erhitzt und dann abgekühlt. Das Gemisch wird zur Trockene eingedampft. Der Rückstand wird mit Silicagel und 3:1 Aethylacetat-Hexan als Eluierungsmittel gereinigt. Man erhält [1α,3β,5Z,7E,24R]-24-fluor-9,10-secocholesta5,7,10(19)-trien-1,3,25-triol (1α,25-

Dihydroxy-24R fluorcholecalciferol), $[\alpha]_D^{23}$ + 68° (c = 0,5, MeOH).

B. Das Ausgangssecocholestan kann wie folgt hergestellt werden:

a) Einer Lösung von 1,2 ml Diisopropylamin in 4 ml Tetrahydrofuran werden bei -30°C 4,5 ml (0,0072 Mol) 1,6M Butyllithium in Hexan zugesetzt. Nach Rühren während einer halben Stunde werden 3,57 g (0,0065 Mol) [1α,3β]-1,3-bis[(Tetrahydro-2H-pyran-2-yl)oxy]-pregn-5-en-21-carbonsäureäthylester in 36 ml Tetrahydrofuran tropfenweise zugesetzt. Das Gemisch wird während einer Stunde bei -30°C gerührt und auf -70°C abgekühlt. Eine Lösung von 3 40 g (0 011 Mol) 4-(1-Aethoxyäthoxy)-3R-fluor-1-jod-4-methylpentan in 6 ml Hexamethylphosphoramid wird tropfenweise zugesetzt. Das Gemisch wird eine Stunde bei -70°C gerührt, auf 25°C erwärmt und während einer Stunde gerührt. Das Gemisch wird mit 9:1 Hexan-Aether verdünnt. Die Lösung wird mit Wasser und gesättigter Kochsalzlösung gewaschen. Die orgamische Phase wird getrocknet, filtriert und zur Trockene eingedampft. Der Rückstand wird durch Säulenchromatographie auf Silicagel gereinigt. Man erhält [1α,3β,24R]-1,3bis[(Tetrahydro-2H-pyran-2-yl)oxy]-25-(1-äthoxyäthoxy) 24-fluor-cholest-5-en-21-carbonsäureäthylester, $[\alpha]_D^{20}$ + 18° (c = 1, $CHCl_3$).

b) Einem Gemisch von 0,34 g (0,0090 Mol) Lithiumaluminiumhydrid und 17 ml Tetrahydrofuran werden bei 0°C 4,26 g [1α,3β,24R]-1,3-bis[(Tetrahydro-2H-pyran-2-yl)oxy]-25-(1-äthoxyäthoxy)-24-fluorcholest-5-en-21-carbonsäureäthylester in 60 ml Tetrahydrofuran zugesetzt. Däs Gemisch wird 1 1/2 Stunden bei 50°C erhitzt, auf 0°C abgekühlt und mit 200 ml Aether verdünnt. Dem Gemisch werden 0,68 ml Wasser und 0,55 ml 10%iger wässriger Natriumhydroxidlösung zugesetzt. Das Gemisch wird eine halbe Stunde bei 25°C gerührt und filtriert. Die Feststoffe werden mit Aether verrieben und filtriert. Nach Eindampfen des Lösungsmittels erhält man [1α,3β,24R]-1,3-bis[(Tetrahydro-2Hpyran-2-y1)oxy]-25-(1-äthoxyäthoxy)-24-fluorcholest-5-en 21-ol, $[\alpha]_D^{25}$ +7° (c = 1,1, $CHCl_3$).

c) Ein Gemisch von 3,99 g (0,0058 Mol) [1α,3β,24R]-1,3bis[(Tetrahydro-2H-pyran-2-y1)oxy]-25-(1-äthoxyäthoxy)24-fluorcholest-5-en-21-ol, 15 ml Pyridin und 2 20 g (0,0115 Mol) p-Toluolsulfonylchlorid wird 18 Stunden bei 0°C gerührt. Das Gemisch wird durch Zusatz von Eis abgekühlt. Das Gemisch wird dann in Wasser geschüttet und mit Methylenchlorid extrahiert. Die organische Phase wird mit 10%iger wässriger Schwefelsäure und gesättigter wässriger Natriumbicarbonatlösung gewaschen. Die organische Phase wird getrocknet, filtriert und zur Trockene eingedampft. Man erhält [1α,3β,24R]-1,3-bis[(Tetrahydro-2H-pyran-2y1)oxy]-25-(1-äthoxyäthoxy)-24-fluorcholest-5-en-21-ol 21-(4-methylbenzolsulfonat), $[\alpha]_D^{20}$ +8° (c = 1, $CHCl_3$).

d) Ein Gemisch von 0,223 g (0,00026 Mol) [1α,3β,24R]1,3-bis[(Tetrahydro-2H-pyran-2-y1)oxy]-25-(1-äthoxyäthoxy)24-fluorcholest-5-en-21-ol-21-(4-methylbenzolsulfonat und 0 150 g (0 0010 Mol) Natriumjodid in 2 ml Aceton wird 18 Stunden auf 50°C erhitzt und dann abgekühlt. Das Gemisch wird in Wasser geschüttet und das Produkt mit Methylenchlorid isoliert. Die organischen Schichten werden mit wässriger Natriumsulfitlösung und gesättigter wässriger Natriumbicarbonatlösung gewaschen. Die organischen Schichten werden getrocknet, filtriert und zur Trockene eingedampft. Man erhält [1α,3β,24R]-1,3-bis[(Tetrahydro-2H-pyran-2yl)oxy]-25-(1-äthoxyäthoxy)-24-fluor-21-iodcholest-5-en.

e)1) Ein Gemisch von 0,710 g (0,0187 Mol) Lithiumaluminiumhydrid, 80 ml Tetrahydrofuran und 4,85 g (0,0057 Mol) [1α,3β,24R]-1,3-bis[(Tetrahydro-2H-pyran-2-y1)oxy]-25(1-äthoxyäthoxy)-24-fluorcholest-5-en-21-ol-21-(4-methylbenzolsulfonat wird unter Rückfluss (60°C) eine Stunde erhitzt und auf 0°C abgekühlt. Das Gemisch wird mit 200 ml Aether verdünnt und es werden 1,4 ml Wasser und 1,1 ml einer 10%igen Natriumhydroxidlösung zugesetzt. Das Gemisch wird eine Stunde gerührt und filtriert. Die Feststoffe werden mit Aether verrieben und filtriert. Die vereinigten Filtrate werden zur Trockene eingedampft und auf Silicagel chromatographiert. Man erhält [1α,3β,24R]-1,3-bis[(Tetrahydro-2H-pyran-2-y1)oxy]-25-(1-äthoxyäthoxy)-24-fluor cholest-5-en, $[\alpha]_D^{20}$ +7° (c = 1, $CHCl_3$).

e)2) In einer Variante wird ein Gemisch von 0 206 g (0,00025 Mol) [1α,3β,24R]-1,3-bis[(Tetrahydro-2H-pyran-2y1)oxy]-25-(1-äthoxyäthoxy)-24-fluor-21-iodcholest-5-en, 0 087 g (0 00030 Mol) tri-n-Butylzinnhydrid und 3 ml Tetrahydrofuran 18 Stunden unter Argon bei 25°C gerührt. Das Gemisch wird zur Trockene eingedampft und der Rückstand chromatographisch auf Silicagel gereinigt. Man erhält [1α,3β,24R]-1,3-bis-[(Tetrahydro-2H-pyran-2-y1)oxy] 25-(1-äthoxyäthoxy)-24-fluorcholest-5-en, $[\alpha]_D^{21}$ +7° (c = 1, $CHCl_3$).

f) Ein Gemisch von 3,56 g (0,0053 Mol) [1α,3β,24R]-1,3bis-[(Tetrahydro-2H-pyran-2-y1)oxy]-25-(1-äthoxyäthoxy)24-fluorcholest-5-en, 140 ml Methanol und 0,58 g p-Toluolsulfonsäuremonohydrat wird 3 Stunden bei 25°C gerührt. Dem Gemisch wird 1 g Natriumbicarbonat zugesetzt. Nach halbstündigem Rühren wird das Gemisch zur Trockene eingedampft. Der Rückstand wird mit Aethylacetat verrieben, filtriert und zur Trockene eingedampft. Der rohe Feststoff wird aus Aethylacetat umkristallisiert. Man erhält [1α, 3β,24R]-24-fluorcholest-5-en-1,3,25-triol, Smp. 156-158°C,

$[\alpha]_D^{21}$ +4° (c = 0 5, MeOH).

g) Ein Gemisch von 1 53 g (0 0035 Mol) [1α 3β,24R]-24fluorcholest-5-en-1 3 25-triol 13 ml Pyridin und 6 ml Acetanhydrid wird eine Stunde bei 0° C und 17 Stunden bei 25° C gerührt. Das Gemisch wird mit 20 ml Methanol bei 0° C verdünnt und zur Trockene eingedampft. Der Rückstand wird in Methylenchlorid gelöst. Die Lösung wird mit 10%iger wässriger Schwefelsäure und gesättigter Natriumbicarbonatlösung gewaschen. Die organische Phase wird getrocknet, filtriert und zur Trockene eingedampft. Man erhält [1α,3β,24R]-24-fluorcholest-5-en-1,3,25-triol-1,3-dia cetat, $[\alpha]_D^{23}$ -7° (c = 1, $CHCl_3$).

h) Ein Gemisch von 0,520 g (0,0010 Mol) [1α,3β,24R]-24fluorcholest-5-en-1,3,25-triol-1,3-diacetat, 0,45 g Natriumbicarbonat, 0,192 g (0,00066 Mol) 1,3-Dibrom-5,5dimethylhydantoin und 25 ml Hexan wird eine Stunde bei Rückfluss (80° C) erhitzt und dann abgekühlt. Das Gemisch wird filtriert und die Feststoffe mit Hexan verrieben und filtriert. Die Filtrate werden zur Trockene eingedampft und man erhält [1α,3β,7ξ,24R]-7-brom-24-fluorcholest-5en-1,3,25-triol-1,3-diacetat.

i) Ein Gemisch von 0,605 g (0,001 Mol) [1α,3β,7 ,24R]7-brom-24-fluorcholest-5-en-1,3,25-triol-1,3-diacetat, 0,6 ml s-Collidin und 18 ml Xylol wird eine halbe Stunde unter Rückfluss (140° C) erhitzt und dann abgekühlt. Das Gemisch wird mit 30 ml Toluol verdünnt. Die Lösung wird mit 10%iger Schwefelsäure und gesättigter Natriumbicarbonatlösung gewaschen. Die organische Phase wird getrocknet, filtriert und zur Trockene eingedampft. Der Rückstand wird durch Säulenchromatographie auf Silicagel gereinigt und man erhält [1α,3β,24R]-24-fluorcholesta 5,7-dien-1,3,25-triol-1,3-diacetat, $[\alpha]_D^{23}$ -21° (c = 0,5, $CHCl_3$).

j) Ein Gemisch von 0,258 g (0,0005 Mol) [1α,3β,24R]-24fluorcholesta-5,7-dien-1,3,25-triol-1,3-diacetat in 40 ml Hexan und 10 ml Tetrahydrofuran wird eine halbe Stunde unter Argon bei -5° C mit einer 450W Hanovia-Hochdruckquecksilberlampe mit Vycorglas-Kühlfinger bestrahlt. Die Lösung wird zur Trockene eingedampft und der Rückstand durch Flüssigchromatographie auf Silicagel mit einem 4:1 n-Hexan-Aethylacetatgemisch als Eluierungsmittel gereinigt. Man erhält [1α,3β,6Z,24R]-24-fluor-9,10-secocholesta-5(10),6,8-trien1,3,25-triol-1,3-diacetat.

k) Eine Lösung von 0,125 g (0,00024 Mol) [1α,3β,6Z,24R]24-fluor-9,10-secocholesta-5(10),6,8-trien-1,3,25-triol1,3-diacetat und 15 ml 1% Kaliumhydroxid in Methanol wird 4 Stunden bei 0° C gerührt. Das Gemisch wird zu pH 7,5 mit Eisessig in Methanol neutralisiert. Die Lösung wird bei 0° C zur Trockene eingedampft. Der Rückstand wird zwischen Aethylacetat und Wasser geteilt. Die organische Phase wird mit gesättigter Kochsalzlösung gewaschen und getrocknet. Das Gemisch wird filtriert und zur Trockene eingedampft. Man erhält [1α,3β,6Z,24R]-24-fluor-9,10-secocholesta-5(10),6,8-trien-1,3,25-triol.

C. Das Zwischenprodukt 4-(1-äthoxyäthoxy)-3R-fluor-1jod-4-methylpentan kann wie folgt hergestellt werden:

a) Einer Lösung von 16,5 g (0,102 Mol)-Diäthylaminoschwefeltrifluorid in 20 ml Methylenchlorid werden bei -70° C tropfenweise 3,50 g (0,034 Mol) (-)-2S-Hydroxy-4butyrolacton in 30 ml Methylenchlorid zugetropft. Das Gemisch wird eine Stunde bei -70° C gerührt und eine Stunde unter Rühren bei 25° C erhitzt. Das Gemisch wird in eine gesättigte Natriumbicarbonatlösung bei 0° C geschüttet. Das Produkt wird mit Methylenchlorid extrahiert. Die organische Phase wird getrocknet, filtriert und zur Trockene eingedampft. Der Rückstand wird durch Säulenchromatographie auf Silicagel gereinigt und man erhält 2R-Fluor-4-butyro lacton, $[\alpha]_D^{22}$ +50° (c = 1, $CHCl_3$).

b) Einer Lösung von 16,8 ml 1,5M atherischem Methyllithium (0,025 Mol) werden bei 0° C 1'05 g (0'010 Mol) 2R-Fluor-4-butyrolacton in 35 ml Aether zugetropft. Der Rückstand wird eine Stunde bei 0° C und eine Stunde bei 25° C gerührt. Dem Gemisch werden 2 ml gesättigter Kochsalzlösung bei 0° C zugefügt. Das Gemisch wird in gesättigter Kochsalzlösung geschüttet und das Produkt mit Aether isoliert. Die Aetherschichten werden getrocknet, filtriert und zur Trockene eingedampft. Man erhält 4-Methyl-3R-fluor 1,4-pentandiol, $[\alpha]_D^{22}$ +39° (c = 1, $CHCl_3$).

c) Einem Gemisch von 0,540 g (0,0040 Mol) 4-Methyl-3Rfluor-1,4-pentandiol und 3 ml Pyridin werden bei 0° C 2 ml Acetanhydrid zugesetzt und das Gemisch wird eine Stunde bei 0° C und eine Stunde bei 25° C gerührt. Das Gemisch wird 10 ml Methanol zugesetzt und die Lösung zur Trockene eingedampft. Man erhält 4-Methyl-3R-fluor-1,4-pentandiol 1-acetat, $[\alpha]_D^{22}$ +49° (c = 0,5, $CHCl_3$).

d) Ein Gemisch von 2,48 g (0,014 Mol) 4-Methyl-3-Rfluor-1,4-pentandiol-1-acetat, 65 ml Aethylvinyläther und 0,25 g p-Toluolsulfonsäuremonohydrat wird eine Stunde bei -70° C gerührt. Dem Gemisch werden 8 ml Triäthylamin zugefügt und es wird zur Trockene eingedampft. Der Rückstand wird in Aether aufgenommen. Die Lösung wird nacheinander mit gesättigter Natriumbicarbonatlösung und gesättigter Kochsalzlösung gewaschen. Die Aetherphase wird getrocknet, filtriert und zur Trockene eingedampft. Man erhält ein Oel, enthaltend 4-(1-Aethoxyäthoxy)-3R-fluor-4-methyl-1pentanol-1-acetat.

e) Einem Gemisch von 0 79 g (0,021 Mol) Lithiumaluminiumhydrid in 40 ml Aether werden bei 0° C 3 48 g (0 014 Mol) 4-(1-Aethoxyäthoxy)-3R-fluor-4-methyl-1-pentanol-1acetat in 100 ml Aether zugetropft. Das Gemisch wird 3 Stunden unter Rückfluss erhitzt und dann auf 0°C abgekühlt. Dem Gemisch werden 1,5 ml Wasser gefolgt von 1,2 ml einer 10%igen Natriumhydroxidlösung zugetropft.

Das Gemisch wird eine halbe Stunde bei 25°C gerührt und filtriert. Die Feststoffe werden mit Aether verrieben und filtriert. Nach Abdampfen des Lösungsmittels und Säulen: chromatographie des Rückstandes auf Silicagel erhält man Y-(1-Aethoxyäthoxy)-3R-fluor-4-methyl-1-pentanol,FIG35/10 +18° (c = 1, $CHCl_3$).

f) Ein Gemisch von 3,34 g (0,016 Mol) 4-(1-Aethoxyäthoxy)-3R-fluor-4-methyl-1-pentanol, 12 ml Pyridin und 4 67 g (0,024 Mol) p-Toluolsulfonylchlorid wird 18 Stunden bei 0°C gerührt. Dem Gemisch wird Eis zugegeben. Dann wird das Gemisch in Wasser geschüttet und mit Methylenchlorid extrahiert. Die organische Phase wird nacheinander mit lOüiger Schwefelsäure und gesättigter Natriumbicarbonatlösung qewaschen. Die organische Phase wird getrocknet, filtriert und zur Trockene eingedampft. Man erhält 4-(1-Aethoxyäthoxy)3R-fluor-4-methyl-1-pentanol-1-(4-methylbenzolsulfonat) in Form eines Oels.

g) Ein Gemisch von 5,42 g (0,015 Mol) 4-(1-Aethoxyäthoxy)-3R-fluor-4-methyl-1-pentanol-1-(4-methylbenzolsulfonat), 70 ml Aceton, 1 ml Diisopropyläthylamin und 30 3 g (0,202 Mol) Natriumjodid wird 18 Stunden bei 25°C gerührt. Das Gemisch wird zur Trockene eingedampft. Der Rückstand wird zwischen einer 5%igen Natriumsulfitlösung und Methylenchlorid aufgeteilt. Die organische Phase wird mit gesättigter Natriumbicarbonatlösung gewaschen, dann getrocknet, filtriert und zur Trockene eingedampft. Man erhält 4-(1-Aethoxyäthoxy)-3R-fluor-1-jod-4-methylpentan,

$[\alpha]_D^{24}$ +43° (c - 1 $CHCl_3$).

D. Das Zwischenprodukt, [1α,3β-1,3-bis[(Tetrahydro-2Hpyran-2-y1)oxy]-pregn-5-en-21-carbonsäureäthylester kann wie folgt hergestellt werden:

a) Ein Gemisch von 0,91 g (0 0030 Mol) 1α,3β-Dihydroxyandrost-5-en-18-on, 15 ml Tetrahydrofuran, 1 26 g (0 015 Mol) 3,4-Dihydro-2H-pyran und 0,028 mg p-Toluolsulfonsäuremonohydrat wird 18 Stunden bei 25°C gerührt. Das Gemisch wird mit Methylenchlorid verdünnt. Die Lösung wird dann mit gesättigter Natriumbicarbonatlösung gewaschen. Die organische Phase wird getrocknet, filtriert und zur Trockene eingedampft. Man erhält [1α,3β]-1,3-bis-[(Tetra hydro-2H-pyran-2-yl)oxy]androst-3-en-17-on $[\alpha]_D^{20}$ +34 3° (c = 1, $CHCl_3$).

b) Einem Gemisch von 1,00 g (0,0021 Mol) [1α 3β]-1 3-bis[(Tetrahydro-2H-pyran-2-yl)oxy]androst-3-en-17-on 1 94 g (0,0087 Mol) Triäthylphosphonacetat und 14 ml Aethanol werden 0,68 g (0,010 Mol) Naüriumäthoxid in 7 ml Aethanol zugesetzt. Das Gemisch wird 18 Stunden unter Rückfluss gerührt und dann abgekühlt. Das Gemisch wird unter vermindertem Druck eingeengt, dann zwischen Wasser und Aether verteilt. Die organische Phase wird mit gesättigter Kochsalzlösung gewaschen. Die organische Phase wird getrocknet, filtriert und zur Trockene eingedampft. Der Rückstand wird auf Silicagel chromatographiert. Man erhält [1α,3β,17(20)E]-1,3-bis[(Tetrahydro-2H-pyran-2-yl)-oxy]pregna 5,17(10)-dien-21-carbonsäureäthylester, $[\alpha]_D^{20}$ -8° (c = 1, $CHCl_3$).

c) Ein Gemisch von 0,32 g (0,00059 Mol) [1α,3β,17(20E]1,3-bis[(Tetrahydro-2H-pyran-2-yl)oxy]pregna-5,17(20)dien-21-carbonsäureäthylester, 0,10 g Platinoxid und 20 ml Aethanol wird 2 Stunden unter einer Atmosphäre Wasserstoff gerührt. Das Gemisch wird durch Diatomeenerde filtriert. Die Feststoffe werden mit Methanol gewaschen. Die vereinigten Filtrate werden zur Trockene eingedampft. Man erhält [1α,3β]-1,3-bis[(Tetrahydro-2H-pyran-2-yl)oxy]pregn-5-en-21 carbonsäureäthylester, $[\alpha]_D^{23}$ -13° (c = 1, $CHCl_3$).

**Beispiel 2**

A. In zu Beispiel 1A analoger Weise wird [1α,3β,6Z,24S]-2Y-Fluor-9,10-secocholesta-5(10),6,8trien-1,3,25-triol zu [1α,3β,5Z,7E,24S]-24-Fluor-9,10-secocholesta-5,7,10(19)-trien-1,3,25-triol (1α,25-dihydroxy-24S-fluorchole calciferol), $[\alpha]_D^{23}$ +45° (c = 0,1, $CH_3OH$) übergeführt.

B. Das Ausgangssecocholestan kann in Analogie zu Beispiel IB ausgehend von [1α,3β]-1,3-bis[(Tetrahydro-2H-pyran-2-yl)oxy]pregn5-en-21-carbonsäureäthylester über die folgenden Zwischenprodukten hergestellt werden:

[1α,3β,24S]-1,3-bis[(Tetrahydro-2H-pyran-2-y1)oxy]25-(1-äthoxyäthoxy)-24-fluorcholest-5-en-21-carbonsäureäthylester,

[1α,3β,24S]-1,3-bis[(Tetrahydro-2H-pyran-2-y1)oxy]25-(1-äthoxyäthoxy)-24-fluorcholest-5-en-21-ol,

[1α,3β,24S]-1,3-bis[(Tetrahydro-2H-pyran-2-y1)oxy]25-(1-äthoxyäthoxy)-24-fluorcholest-5-en-21-ol 21-(4methylbenzolsulfonat),

[1α,3β,24S]-1,3-bis[(Tetrahydro-2H-pyran-2-y1)oxy]25-(1-äthoxyäthoxy)-24-fluor-21-iodcholest-5-en,

[1α,3β,24S]-1,3-bis[(Tetrahydro-2H-pyran-2-y1)oxy]25-(1-äthoxyäthoxy)-24-fluorcholest-5-en,

[1α,3β,24S]-24-Fluorcholest-5-en-1,3,25-triol,

[1α,3β,24S]-24-Fluorcholest-5-en-1,3,25-triol-1,3diacetat,

[1α'3β'7ξ,24S]-7-Brom-24-fluorcholest-5-en-1,3,25triol-1,3-diacetat,

[1α,3β,24S]-24-Fluorcholesta-5,7-dien-1,3,25-triol1,3-diacetat,

[1α,3β,6Z,24S]-24-Fluor-9,10-secocholesta-5(10),6,8trien-1,3,25-triol-1,3-diacetat.

C. Das Zwischenprodukt 4-(1-Aethoxyäthoxy)-3S-fluor-1 jod-4-methylpentan, $[\alpha]_D^{22}$ -38° (c = 1, $CHCl_3$) kann in Ana- logie zu Beispiel 1C ausgehend von (+)-2R-Hydroxy-4-butyrolacton über die folgenden Zwischenprodukte hergestellt werden:

2S-Fluor-4-butyrolacton, $[\alpha]^{23}$ -51° (c = 0,9, $CHCl_3$),

4-Methyl-3S-flour-1,4-pentandiol,$[\alpha]^{22}$ -40° (c= 1, $CHCl_3$),

4-Methyl-3S-fluor-1,4-pentandiol-1-acetat, $[\alpha]_D^{22}$ -49° (c = 0,5, $CHCl_3$), 1-acetat,

4-(1-Aethoxyäthoxy)-3S-fluor-4-methyl-1-pentanol, $[\alpha]_D^{22}$ -16° ($_c$ - 1 $CHC_1$ )

4-(1-Aethoxyäthoxy)-3S-fluor-4-methyl-1-pentanol1-(4-methylbenzolsulfonat).

**Beispiel 3**

Kapseln werden hergestellt durch Auflösung der Bestandteile 1, 3 und 4 im Bestandteil 2 unter Stickstoff und Abfüllen in Kapseln.

| Bestandteile | mg/Kapsel | | | |
|---|---|---|---|---|
| 1. 1α,25-Dihydroxy-24R-fluorcholecalciferol | 0,00010 | 0,00025 | 0,00050 | 0,00100 |
| 2. Polyäthylenglykol | 200,00 | 200,00 | 200,0 | 200,00 |
| 3. Butyliertes Hydroxyanisol | 0,100 | 0,100 | 0,100 | 0,100 |
| 4. Ascorbylpalmitat | 1,00 | 1,00 | 1,00 | 1,00 |

**Beispiel 4**

Eine Lösung zur intramuskulären Injektion wird durch Lösung der Bestandteile 1 und 2 unter Stickstoff hergestellt.

| | | | |
|---|---|---|---|
| 1. 1α,25-Dihydroxy-24R-fluorcholecalciferol | 0,10 mg | 0,50 mg | 1,00 mg |
| 2. 95% Aethanol -5% Wasser | 2,00 ml | 3,00 ml | 3,00 ml |

**Patentansprüche** für die Vertragsstaaten BE, CH, DE, PR, GB, IT, LI, LU, NL, SE

1. Ein Cholecalciferolderivat der Formel

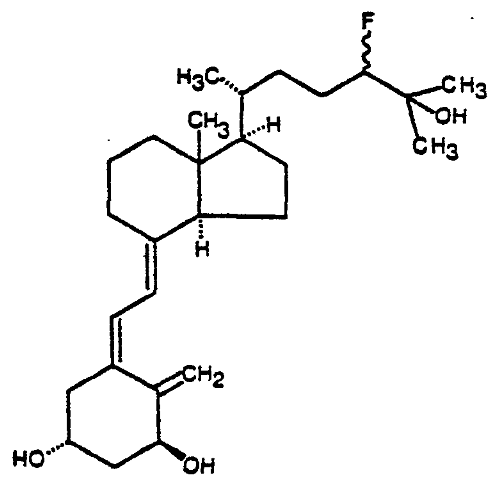

I

2. Die Verbindung nach Anspruch 1 1α 25-Dihydroxy(24R or 24S)-fluorcholecalciferol.

3. Eine Verbindung nach Anspruch 1 oder 2 als pharmazeutischer Wirkstoff.

4. Eine Verbindung nach Anspruch-1 oder 2 als Wirkstoff zur Erhöhung des Calcium- und Phosphatspiegels im Serum und zur Anlagerung dieser Mineralien in den Knochen.

5. Ein Verfahren zur Herstellung einer Verbindung nach Anspruch 1, dadurch gekennzeichnet, dass man ein Precholecalciferolderivat der Formel

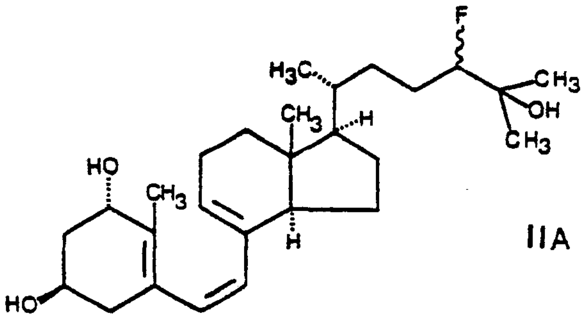

IIA

thermisch isomerisiert.

6. Arzneimittel enthaltend eine Verbindung der Formel I nach Anspruch 1.

7. Arzneimittel nach Anspruch 6 zur Erhöhung des Calcium- und Phosphatspiegels im Serum und zur Anlagerung dieser Mineralien in den Knochen.

8 . Ein PrechoLecaLciferolderivat der Formel

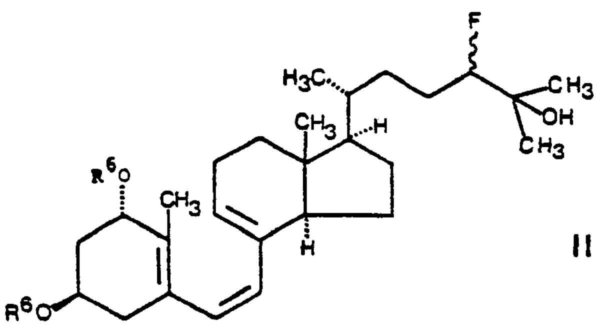

II

worin $R^6$ Wasserstoff oder Niederalkanoyl darstellt.

9 . Eine Verbindung aus der Gruppe der folgenden

[1α,3β,6Z,(24R oder 24S)]-24-Fluor-9,10-secocholecta5(10),6,8-trien-1,3,25-triol-1,3-diacetat und

[1α,3β,6Z,(24R oder 24S)]-24-Fluor-9,10-secocholesta5(10),6,8-trien-1,3,25-triol.

10 . Ein Cholestenderivat der Formel

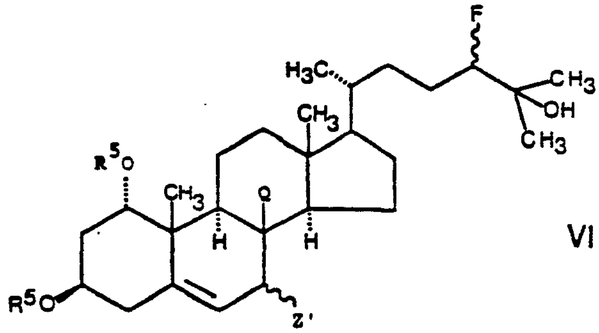

VI

worin $R^5$ Niederalkanoyl, Q Wasserstoff und Z' Wasserstoff, Jod, Brom oder Chlor, oder Q und Z' zusammen eine zusätzliche 7,8-Bindung darstellen.

11. Eine Verbindung aus der Gruppe der folgenden

[1α,3β,(24R oder 24S)]-24-Fluorcholest-5-en-1,3,25triol-1,3-diacetat,

[1α 3β 7ξ (24R oder 24S)]-7-Brom-24-fluorcholest-5en-1,3,25-triol-1,3-diacetat und

[1α,3β,(24R oder 24S)]-24-Fluorcholesta-5,7-dien1,3,25-triol-1,3-diacetat.

12. Ein Cholestenderivat der Formel

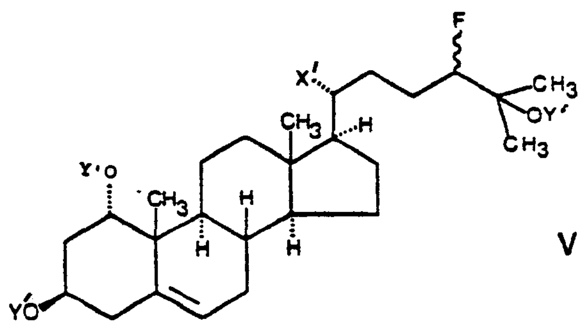

V

worin Y' Wasserstoff oder eine Gruppe Y und X' Methyl, oder Y' eine Gruppe Y und X' eine Gruppe $-CH_2X$, $-CH_2OH$ oder $COOR^4$; Y eine Gruppe der Formel

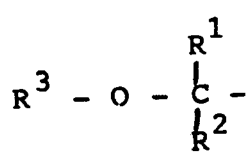

worin $R^1$ Wasserstoff oder Niederalkyl, $R^2$ und $R^3$ Niederalkyl oder $R^2$ und $R^3$ zusammen $C_{3-6}$-Alkylen, X Jod, Brom, Chlor, Niederalkylsulfonyloxy, Phenylsulfonyloxy oder substituiertes Phenylsulfonyloxy und $R^4$ Niederalkyl, Phenyl oder substituiertes Phenyl

darstellen.

13. Eine Verbindung aus der Gruppe der folgenden

[1α,3β,(24R oder 24S)]-1,3-bis[(Tetrahydro-2H-pyranyl)-oxy]-25-(1-äthoxyathoxy)-24-fluorcholest 21-carbonsäureäthylester,

[1α 3β,(24R oder 24S)]-1,3-bis[(Tetrahydro-2H-pyran, 2-y1)oxy]-25-(1-äthoxyäthoxy)-24-fluorcholest-5-en-21-ol,

[1α 3β,(24R oder 24S)]-1,3-bis[(Tetrahydro-2H-pyran2-yl)-oxy]-25-(1-äthoxyäthoxy)-24-fluorcholest-5-en-21-ol21-(4-methylbenzolsulfonat),

[1α,3β,(24R oder 24S)]-1,3-bis[(Tetrahydro-2H-pyran2-yl)-oxy]-25-(1-äthoxyäthoxy)-24-fluor-21-jodcholest-5en,

[1α,3β,(24R oder 24S)]-1, 3-bis[(Tetrahydro-2H-pyran2-yl)-oxy]-25-(1-äthoxyäthoxy)-24-fluorcholest-5-en und

[1α,3β,(24R oder 24S)]-24-Fluorcholest-5-en-1,3,25triol.

**Patentansprüche** für den Vertragsstaat: AT

1. Verfahren zur Herstellung eines Cholecalciferolderivats der Formel

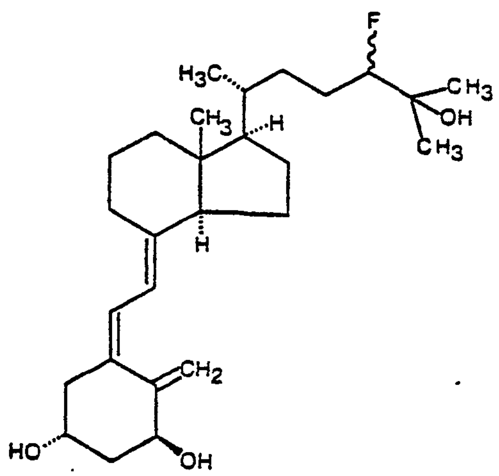

I

dadurch gekennzeichnet, dass man ein Precholecalciferolderivat der Formel

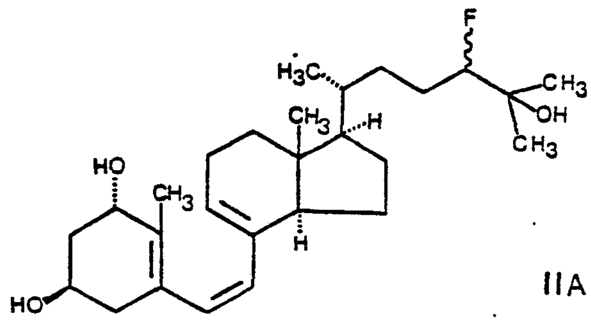

IIA

thermisch isomerisiert.

2. Verfahren nach Anspruch 1, dadurch gekennzeichnet, dass man 1α,25-Dihydroxy-(24R oder 24S)-fluorcholecalciferol herstellt.

**Claims** for the contracting states: BE, CH, DE, FR, GB, IT, LI, LU, NL, SE

1. A cholecalciferol derivative of the formula

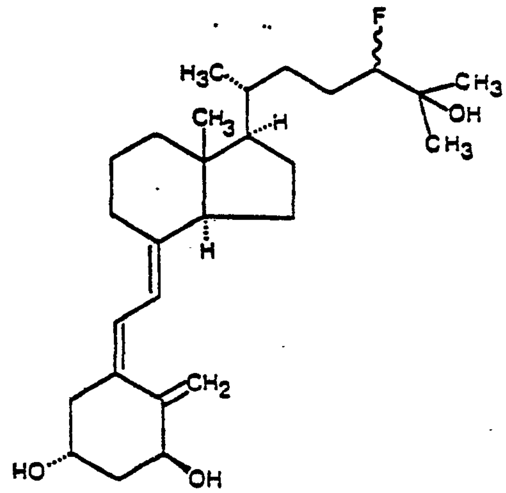

2. The compound according to claim 1, 1α,25- -dihydroxy-(24R or 24S)-fluorocholecalciferol.

3. A compound according to claim 1 or 2 as a pharmaceutically active substance.

4. A compound according to claim 1 or 2 as an active substance for increasing the calcium and phosphate.level in serum and for accumulating these minerals in bones.

5. A process for the manufacture of a

compound according to claim 1, characterized by thermally isomerizing a precholecalciferol derivative of the formula

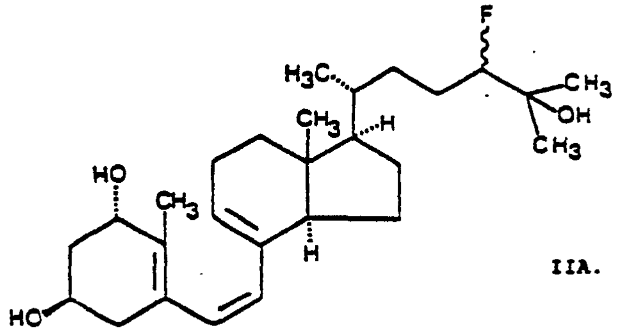

IIA.

6. A medicament containing acompound of formula I according to claim 1.

7. A medicament according to claim 6 for increasing the calcium and phosphate level in serum and for accumulating these minerals in bones.

8. A precholecalciferol derivative of the formula

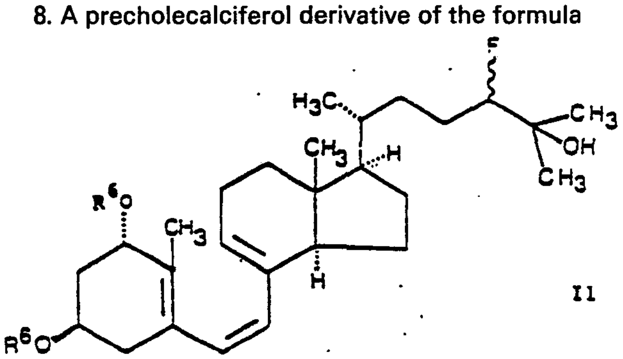

II

wherein $R^6$ represents hydrogen or lower alkanoyl.

9. A compound from the following group %4A1α,3β,6Z,(24R or 24S)]-24-fluoro-9,10-secocholecta-5(10),6,8-triene-1,3,25-triol-1,3-diacetate and [1α,3β,6Z,(24R or 24S)]-24-fluoro-9,10-secocholesta-5(10),6,8-triene-1,3,25-triol.

10. A cholestene derivative of the formula

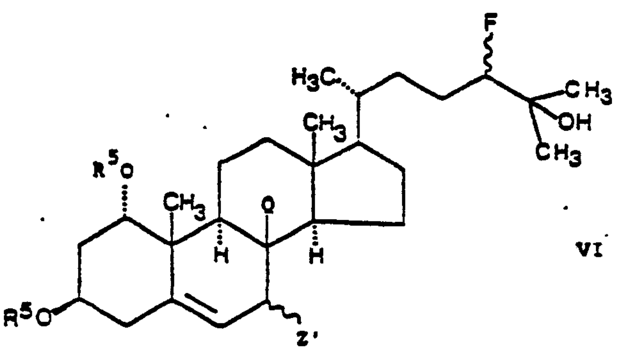

VI

wherein $R^5$ represents lower alkanoyl, Q represents hydrogen and Z' represents hydrogen, iodine, bromine or chlorine, or Q and Z' together represent an additional 7,8-bond.

11. A compound from the following group [1α,3β,(24R or 24S)]-24-fluorocholest-5-ene-1,3,25-triol-1,3-diacetate, (1α,3β,7ξ,(24R or 24S))-7-bromo-24-fluorocholest-5-ene-1,3,25-triol-1,3-diacetate and [1α,3β,(24R or 24S)]-24-fluorocholesta-5,7-diene-1,3,25-triol-1,3-diacetate.

12. A cholestene derivative of the formula

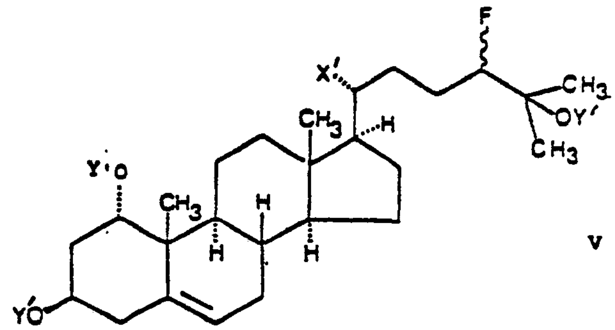

V

wherein Y' represents hydrogen or a group Y and X' represents methyl, or Y' represents a group Y and X' represents a group $-CH_2X$, $-CH_2OH$ or $COOR^4$; Y represents a group of the formula,

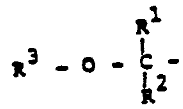

wherein $R^1$ represents hydrogen or lower alkyl, $R^2$ and $R^3$ represent lower alkyl or $R^2$ and $R^3$ together represent $C_{3-6}$-alkylene, X represents iodine, bromine, chlorine, lower alkylsulphonyloxy, phenylsulphonyloxy or substituted phenylsulphonyloxy and $R^4$ represents lower alkyl, phenyl or substituted phenyl.

13. A compound from the following group [1α,3β,(24R or 248)]-1,3-bis[(tetrahydro-2H- -pyranyl)-oxy]-25-(1-ethoxyethoxy)-24-fluorocholest-5-ene-21-carboxylic acid ethyl ester,

[1α,3β,(24R or 24S)-1,3-bis[(tetrahydro-2H-pyran-2-yl)-oxy]-25-(1-ethoxyethoxy)-24-fluorocholest-5-en-21-ol,

[1α,3β,(24R or 248)]-l,3,bis[(tetrahydro-2H-pyran-2-yl)-oxy]-25-(1-ethoxyethoxy)-24-fluorocholest-5-en-21-ol-21-(4-methylbenzenesulphonate),

[1α,3β,(24R or 24S)]-1,3-bis[(tetrahydro-2H-pyran-2-yl)-oxy)-25-(1-ethoxyethoxy)-24-fluoro-21-iodocholest-5-ene,

[1α,3β,(24R or 24S)]-1,3-bis[(tetrahydro-2H-pyran-2-yl)-oxy]-25-(1-ethoxyethoxy)-24-fluorocholest-5-ene and

[1α,3β,(24R or 24S)]-24-fluorocholest-5-ene-1,3,25-triol.

**Claims** for the contracting state :AT

1. A process for the manufacture of a cholecalciferol derivative of the formula

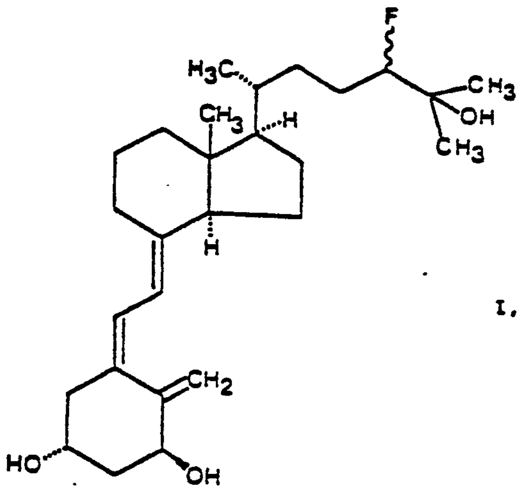

I,

characterized by thermally isomerizing a precholecalciferol derivative of the formula

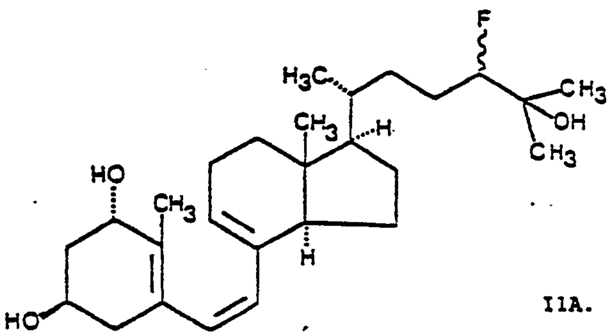

IIA.

2. A process according to claim 1, characterized in that 1α,25-dihydroxy-(24R or 24S)-fluorocholecalciferol is manufactured.

**Revendications** pour les Etats contractants : BE, CH, DE, FR, GB, IT, LI, LU, NL, SE

1.- Un dérivé du cholécalciférol de formule :

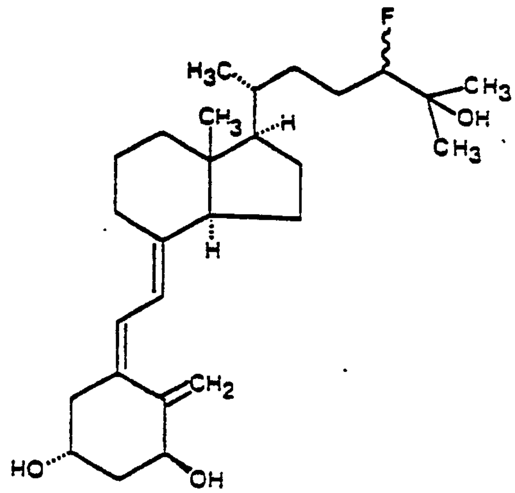

I

2.- Le composé selon la revendication 1, 1α,25-dihydroxy (24R ou 24S)-fluorocholécalciférol.

3.- Un composé selon la revendication 1 ou 2 en tant que substance active pharmaceutique.

4.- Un composé selon la revendication 1 ou 2 en tant que substance active servant à accroitre la teneur en calcium et en phosphate du sérum et à provoquer la fixation de ces minéraux dans les os.

5.- Un procédé de préparation d'un composé selon la revendication 1, caractérisé en ce que l'on soumet un dériVé de préchlolécalciférol de formule :

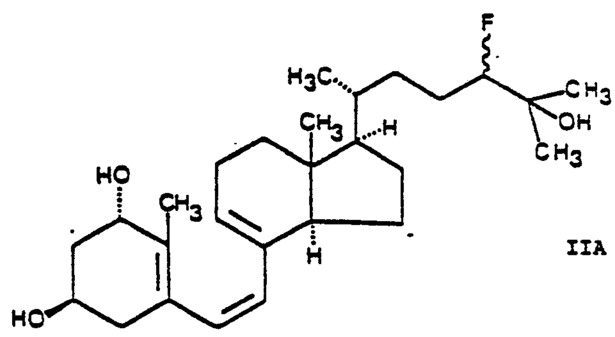

IIA

à isomération à la chaleur.

6.- Médicament contenant un composé de formule I selon la revendication 1.

7.- Médicament selon la revendication 6, servant à accroitre la teneur en calcium et en phosphate du sérum et à provoquer la fixation de ces minéraux dans les os.

8.- Un dérivé de précholécalciférol de formule :

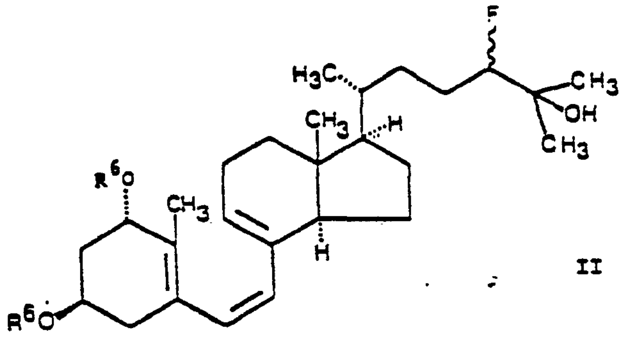

II

dans laquelle $R^6$ représente l'hydrogène ou un groupe alcanoyle inférieur.

9.- Un composé du groupe des suivants :

. 1,3-diacétate du [1α,3β,6Z,(24R ou 24S)]-24-fluoro-9,10-sécocholesta-5(10),6,8-triène1,3,25-triol ; et

. [1α,3β,6Z,(24R ou 24S)]-24-fluoro- 9,10-sécocholesta-5(10),6,8-triène-1,3,25-triol.

10.- Un dérivé du cholestène de formule :

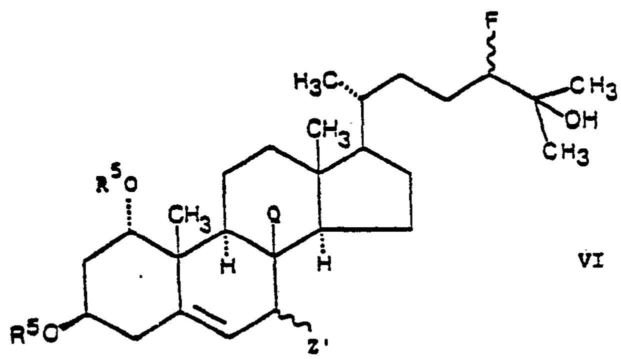

VI

dans laquelle $R^5$ représente un groupe alcanoyle inférieur, Q représente l'hydrogène et Z' représente l'hydrogène, l'iode, le brome ou le chlore, ou bien Q et Z' représentent ensemble une liaison supplémentaire en 7,8.

11.- Un composé du groupe des suivants :

. 1,3-diacétate du [1α,3β,(24R ou 24S)]-24-fluorocholesta-5-ène-1,3,25-triol ;

. 1,3-diacétate du [1α,3β,7ε ,(24R ou 24S)]- 7-bromo-24-fluorocholesta-5-ène-1,3,25-triol ; et

. 1,3-diacétate du [1α,3β,(24R ou 24S)]-24-fluorocholesta-5,7-diène-1,3,25-triol.

12.- Un dérivé du cholestène de formule :

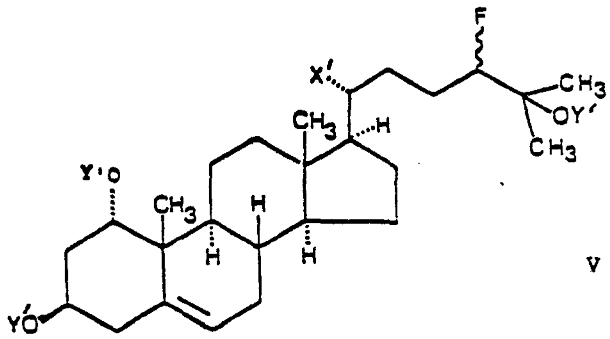

V

dans laquelle Y' représente l'hydrogène ou un groupe Y et X' représente un groupe méthyle, ou bien Y' représente un groupe Y et X' un groupe $-CH_2X$, $-CH_2OH$ ou $COOR^4$ ; Y représente un groupe de formule:

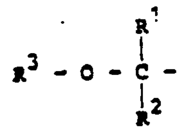

dans laquelle $R^1$ représente l'hydrogène ou un groupe alkyle inférieur, $R^2$ et $R^3$ représentent des groupes alkyle inférieurs ou bien $R^2$ et $R^3$ forment ensemble un groupe alkylène en $C_3$-$C_6$, X représente l'iode, le brome, le chlore, un groupe alkylsulfonyloxy inférieur, phénylsulfonyloxy ou phénylsulfonyloxy substitué et $R^4$ représente un groupe alkyle inférieur, phényle ou phényle substitué.

13.- Un composé du groupe des suivants :

. [1α,3β,(24R ou 24S)]-1,3-bis-[(tétra- hydro-2H-pyrannyl)-oxy]-25-(1-éthoxyéthoxy)-24-fluorocholesta-5-ène-21-carboxylate d'éthyle ;

. [1α'3β,(24R ou 24S)]-1,3-bis-[(tétra- hydro-2H-pyranne-2-yl)-oxy]-25-(1-éthoxyéthoxy)-24fluorocholesta-5-ène-21-ol ;

. 21-(4-méthylbenzènesulfonate) du [1α,3β,(24R ou 24S)]-1,3-bis-[(tétrahydro-2H-pyranne2-yl)-oxy]-25-(1-éthoxyéthoxy)-24-fluorocholesta-5ène-21-ol ,;

. [1α,3β,(24R ou 24S)] -1,3-bis-[(tétra- hydro-2H-pyranne-2-yl)-oxy]-25-(1-éthoxyéthoxy)-24fluoro-21-iodocholesta-5-ène ;

. [1α,3β,(24R ou 24S)]-1,3-bis-[(tétra- hydro-2H-pyranne-2-yl)-oxy] -25-(1-éthoxyéthoxy)-24fluorocholesta-5-ène ; et

. [1α,3β,(24R ou 24S)]-24-fluorocholesta- 5-ène-1,3,25-triol.

**REVENATDICATIONS** pour l'Etat contractant: AT

1.- Procédé de préparation d'un dérivé du cholécalciférol de formule:

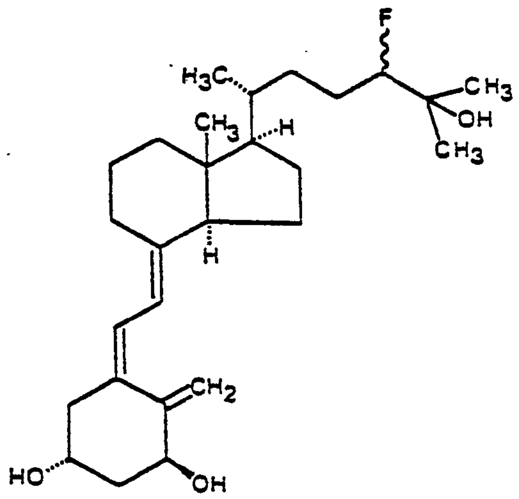

I

caractérisé en ce que l'on soumet un dérivé de précholécalciférol de formule:

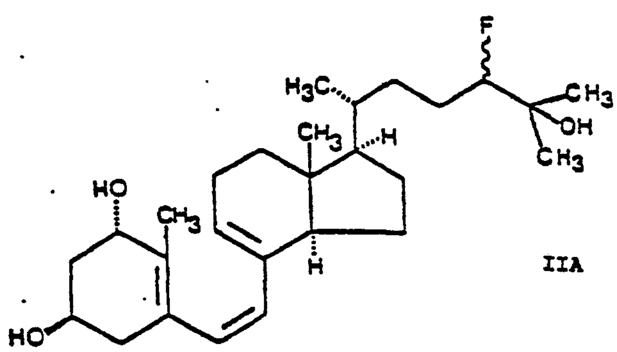

IIA

à isomérisation à la chaleur.

2.- Procédé selon la revendication 1, caractérisé en ce que l'on prépare le 1α,25-dihydroxy-(24R ou 24S)-fluorocholécalciférol.